# EUROPÄISCHE PATENTANMELDUNG

(11) **EP 2 764 872 A1**
(43) Veröffentlichungstag der Anmeldung: **13.08.2014**
(21) Anmeldenummer: 14157534.0
(22) Anmeldetag: 19.08.2009
(51) Int. Cl.: A61K 38/52, A61K 33/32, A61P 1/14

(54) **Behandlung von Fruktose-Malabsorption**

(30) Priorität: 04.09.2008 EP 08450128; 04.09.2008 AT 13802008
(62) Teilanmeldung aus: 09775597.9
(71) Anmelder: Sciotec Diagnostic Technologies GmbH, 3430 Tulln (AT)
(72) Erfinder: Missbichler, Albert, A-1210 Wien (AT)
(74) Vertreter: Fleuchaus, Andrea

(57) **Zusammenfassung**

Die vorliegende Erfindung betrifft die Zusammensetzung umfassend kristalline Xylose-Isomerase (EC 5.3.1.5) und zumindest ein Salz eines Metall- und/oder Erdalkalimetalls.

## Beschreibung

Die vorliegende Erfindung betrifft eine Zusammensetzung zur Behandlung von Fruktose-Malabsorption.

Fruktose (Fruchtzucker, oft auch veraltet Lävulose) gehört als Monosaccharid (Einfachzucker) zu den Kohlehydraten. Fruktose ist eine sehr verbreitete Zuckerform in der menschlichen Ernährung.

Fruktose kommt als freie Hexose, im Haushaltszucker (Rohrund Rübenzucker) als Disaccharid Saccharose gebunden an Glukose und in polymerisierter Form als unverdaubare Fruktane vor. Auf Grund ihrer um etwa 20% höheren Süsskraft gegenüber normalem Zucker und günstiger Transportmöglichkeiten wird freie Fruktose zunehmend im Lebensmittelbereich eingesetzt.

Im Gegensatz zu Glukose wird Fruktose im Darm nicht aktiv aufgenommen, sondern wird deutlich langsamer über spezielle Proteine passiv resorbiert. Knapp die Hälfte der Bevölkerung ist nicht fähig, mehr als 25 g Fruktose pro Tag zu resorbieren. Der durchschnittliche tägliche Konsum liegt jedoch zwischen 11 g und 54 g pro Tag. Dabei ist wichtig anzumerken, dass der Großteil der Fruktose über Softdrinks zugeführt wird, die eine zunehmende Bedeutung in der durchschnittlichen Nahrungsaufnahme haben. Der zunehmende Einsatz von HFCS ("high fructose corn syrups") als Süssstoff verstärkt das Problem zusätzlich.

Als Konsequenz dieser Malabsorption entstehen eine Störung des osmotischen Gleichgewichts und zusätzlich ein rascher Abbau durch die Bakterien im Dickdarm. Dies führt einerseits zu störender Gasbildung im Bauch, einer Beeinträchtigung der Darmmotilität und mittelfristig zu einer Veränderung der Bakterienpopulation. Als Folge kann sich ein klinisch manifestes Reizdarmsyndrom ergeben.

Die Diagnose einer Fruktose-Malabsorption erfolgt gemäß dem Stand der Technik mit einer Fruktose-Provokation und anschließender Bestimmung des H₂-Gehalts in der Atemluft. Die Spezifität dieses Tests liegt weit unter 50%.

Als Therapie steht bislang ausschließlich eine diätetische Behandlung zur Verfügung, die aber aufgrund des oben geschilderten breiten Einsatzes von Fruktose für den Anwender sehr schwierig einzuhalten ist. Zusätzlich führt die Vermeidung von Obst zu Mangelerscheinungen, welche wiederum kompensiert werden müssen.

Die Aufnahme von Kohlehydraten im Dünndarm basiert auf der hydrolytischen Spaltung durch Hydrolasen im Darmlumen und an der Darmschleimhaut in Hexose-Monosaccharide, Glukose, Galaktose und Fruktose, welche dann durch das Darmepithel resorbiert werden. Diese Resorption wird zum überwiegenden Anteil von drei Transportproteinen bewerkstelligt: SGLT1, GLUT5 und GLUT2.

SGLT1 (Natrium/Glukose co-Transporter) arbeitet im Bürstensaum des Dünndarmepithels. SGLT1 transportiert Glukose und Galaktose gegen einen Konzentrationsgradienten, speziell bei niedriger Glukosekonzentration im Darmlumen.

GLUT5 ist spezifisch für Fruktose und ein so genannter fakultativer Transporter. Damit ist GLUT5 stark von einem Konzentrationsgefälle zwischen Darmlumen und Blutkreislauf abhängig. GLUT5 ist in der gesamten Dünndarmwand vorhanden.

GLUT2 schließlich ist ein niedrigaffiner fakultativer Transporter von Glukose, Fruktose und Galaktose. GLUT2 wird offenbar bei SGLT1-Aktivität rasch und reversibel in die Darmwand eingebaut. Die Aktivität von GLUT2 ist von multiplen Faktoren abhängig und wird daher oft als "diffusional pathway" bezeichnet.

Fruktose Malabsorption kann zu verschiedensten Konsequenzen führen.

Als kleine Moleküle aggregieren Fruktose und Fruktane große Mengen an Wasser um sich und transportieren dieses in den distalen Dünndarm und schließlich in den Dickdarm. Dies bewirkt eine Beschleunigung des Darmtransfers, der Effekt wird bei Abführmitteln angewandt.

In den Dickdarm eingebrachte Fruktose wird von den im Wirt vorhandenen Bakterien rasch in kurzkettige Fettsäuren umgewandelt. Dabei entstehen große Mengen an Wasserstoff, CO₂ und manchmal auch Methan. Die kurzkettigen Fettsäuren beeinflussen den pH-Wert des Darmes und sorgen auch für eine höhere Motilität.

Manche Bakterien verwenden Fruktose zur Bildung von Fruktanen, die als Adhäsionsfaktoren an die Darmwand dienen. Der Einfluss von diesen dann adhärenten Bakterien ist vielfältig und umstritten. Bei Ratten wurde eine gesteigerte Epithel-Proliferation und exzessive Mucin-Ausschüttung beobachtet, dies wird normalerweise mit einer Schleimhautirritation in Verbidung gebracht.

Zusätzlich wird Fruktose-Malabsorption auch mit Depressionen in Zusammenhang gebracht, da die im Blutkreislauf vorhandene Menge an Tryptophan, dem Prekursor von Serotonin, beeinflusst wird.

Der Einfluss von Fruktose-Malabsorption auf die Entwicklung von gastrointestinalen Beschwerden wurde erstmals 1978 erkannt (Andersson DE, Nygren A: Acta Med Scand 1978; 203:87-92). Dennoch - und wohl auch aufgrund einer fehlenden spezifischen und sensitiven Diagnosemethode - ist Fruktose-Malabsorption bislang generell als Krankheit nicht anerkannt. Dies ist auch deshalb verständlich, weil es unterschiedlich starke Absorptionsstörungen gibt und der Übergang von "normal" zu "pathologisch" sehr starken personenspezifischen Schwankungen unterworfen ist.

Fruktose stellt nicht nur ein Problem bei Fruktose-Malabsorption dar, sondern auch bei der hereditären Fruktose-Intoleranz, wobei beide Krankheitsbilder in der Literatur zuweilen als Fruktose-Intoleranz subsummiert werden. Unverträglichkeit von Fruchtzucker kommt in einer Häufigkeit von ca. 1:20.000 vor. Es handelt sich dabei um eine autosomal-rezessive erbliche Störung des Fruktosestoffwechsels, bei der Fruktose nicht oder nicht in ausreichenden Mengen abgebaut werden kann. Hieraus resultiert ein erhöhter Fruchtzuckergehalt in den Zellen mit toxischer Wirkung, der seinerseits die Verstoffwechselung der Glukose stört. Folge davon ist eine Unterzuckerung (Hypoglykämien).

Fruktose-Malabsorption kann nur durch strenge Fruktose-arme bzw. -lose Diäten behandelt werden. Durch eine einseitige Ernährung, bei der insbesondere auf Lebensmittel wie Obst, Obstsäfte usw. verzichtet werden muss, kommt es häufig zu Mangelerscheinungen, die auf den Gesundheitszustand der Patienten einen negativen Effekt ausüben. Um etwaigen Mängeln vorzubeugen müssen daher neben einer strengen Diät auch zusätzliche Produkte wie z.B. Vitaminpräparate von den Patienten aufgenommen werden. Es muss also im Interesse aller Betroffenen aber auch des Gesundheitssystems sein, eine effiziente und für breite Bevölkerungsschichten leistbare Therapieform zur Verfügung zu stellen.

In der WO 2007/057749 werden Präparationen beschrieben, die auf dem Einsatz von 5-D-Fruktose-Dehydrogenase (EC 1.1.1.124; FDH) basieren. Dieses Enzym verändert Fruktose dahingehend, dass es von Bakterien im Gastro-Intestinal-Trakt nicht mehr als Substrat verwendet werden kann und daher auch keine Beschwerden auslösen kann. Zur Unterstützung der Wirkung von FDH wird die Zugabe von Xylose-Isomerase vorgeschlagen.

In der DE 102006013624 wird Xylose-Isomerase allgemein als Mittel zur Umwandlung von Fruktose in Glukose beschrieben.

Es ist Aufgabe der vorliegenden Erfindung Mittel zur Verfügung zu stellen, welche im Falle von Fruktose-Malabsorption das Vordringen von Fruktose in den Dickdarm zu verhindern vermögen, um damit auch die dabei entstehenden Krankheitssymptome zu unterbinden bzw. signifikant zu reduzieren. Dadurch kann den Patienten, die an einem derartigen Leiden erkrankt sind, ermöglicht werden, ein weitgehend "normales" Leben zu führen, bei dem auf Fruktose-hältige Lebensmittel nicht verzichtet werden muss.

Die vorliegende Erfindung betrifft eine Zusammensetzung umfassend kristalline Xylose-Isomerase (EC 5.3.1.5) und zumindest ein Salz eines Metall- und/oder Erdalkalimetalls.

Es hat sich überraschender Weise herausgestellt, dass Xylose-Isomerase in kristalliner Form und in Anwesenheit von Salzen von Metall und/oder Erdalkalimetallen eine hohe Aktivität gegenüber in herkömmlicher Weise hergestellter Xylose-Isomerase aufweist. Die kristalline Form der Xylose-Isomerase hat auch den Vorteil, dass das Enzym vor beispielsweise Säureeinwirkung im Magen und Proteasen geschützt ist, wenn die kristalline Xylose-Isomerase quervernetzt wird. Die Quervernetzung kann durch etablierte Verfahren erreicht werden (z.B. Vallejo-Becerra et al. (J Agric Food Chem. 2008, Feb. 07; 56(4): 1392 - 1397) oder Wenzel et al. (FEBS Lett. 1991, March 11; 280(1): 147 - 151) Dadurch könnte Xylose-Isomerase bei einer oralen Verabreichung gegebenenfalls ohne oder mit einer reduzierten Magensäure-resistenten Beschichtung verabreicht werden.

Die Xylose-Isomerase bzw. die Zusammensetzung liegt vorzugsweise als getrocknetes feinkörniges Pulver vor, das vorzugsweise ohne die Zugabe oder in Gegenwart von Metallionen als Kofaktoren kristallisiert worden ist, um eine rasche Bioverfügbarkeit und hohe spezifische Aktivität sicherzustellen. Die Kristalle der Xylose-Isomerase können durch eine Mühle feinkörnig gemahlen werden. Diese Art der Herstellung bewirkt eine maximale Aktivität im physiologischen Milieu des Darms und eine schnelle Freisetzung aufgrund einer sehr guten Löslichkeit im Darmlumen.

Mit Hilfe der erfindungsgemäßen Präparation der Xylose-Iomerase konnte überraschenderweise die Aktivität und Bioverfügbarkeit um ein Vielfaches gesteigert werden. Bei Probanden konnte gezeigt werden, dass diese hohe Aktivität und Wirksamkeit ein Vordringen der Fruktose in den Dickdarm wesentlich bzw. völlig verhindert. Durch die rascher erfolgende Resorption von Glukose im Darmbereich wird der Gleichgewichtsreaktion (Fruktose <-> Glukose) ständig Glukose entzogen, so dass Fruktose über die Zeit abgebaut wird. Dadurch wird verhindert, dass überschüssige Fruktose im Darmbereich verbleibt und zu den bekannten Gesundheitsstörungen aufgrund von Fruktose-Malabsorption und in weiterer Folge zu Symptomen der Fruktose-Malabsorption führt.

Xylose-Isomerase wird großtechnisch in der Lebensmittelindustrie zur Herstellung von Fruktose aus Glukose verwendet, um einen höheren Süßungsgrad zu erreichen. Unter den Umgebungsparametern im Dünndarm (d.h. Entzug von Glukose aus der Gleichgewichstreaktion) wird die Aktivität der erfindungsgemäß hergestellten Xylose-Isomerase in die umgekehrte Richtung gedrängt: das Enzym isomerisiert Fruktose zu Glukose. Industriell eingesetztes Enzym enthält aber große Mengen an Störsubstanzen, die die Aktivität des Enzymes entscheidend inhibieren, insbesondere Sorbitol. Im Gegensatz zum industriell eingesetzten Enzym ist der Gehalt der erfindungsgemäßen kristallinen Xylose-Isomerase an Sorbitol <1%. Bedingt durch einen Gehalt von Sorbitol unter 1% kann mit der erfindungsgemäßen Zusammensetzung eine nennenswerte Aktivitätssteigerung erreicht werden. Die mit der Abwesenheit von Sorbitol zu befürchtende Stabilitätsbeeinträchtigung zeigte sich überraschender Weise als nicht geeignet, die bessere Funktionalität der Erfindung entscheidend zu beeinträchtigen.

Unterstützend zur Xylose-Isomerase kann auch Mannose-Isomerase zur Umwandlung von Fruktose in Mannose verwendet werden. Diese wird ebenfalls im Dünndarm wie oben skizziert resorbiert und damit dem Reaktionsgleichgewicht entzogen. Die Kristallisation der Xylose-Isomerase erfolgt nach Verfahren, die im Stand der Technik hinreichend bekannt sind (Suzuki Y et al., J Phys Chem B. 109(8) 2005: 3222-6; Dunlop KV and Hazes B, Acta Crystallogr D Biol Crystallogr. 61 2005: 1041-8; Vilonen KM et al., Biotechnol. Prog. 20(5) 2004: 1555-60; Ramagopal UA et al., Acta Crystallogr D Biol Crystallogr. 59 2003: 868-75.

Um eine hohe Enzymaktivität im Darmbereich, insbesondere im Dünndarm, zu erzielen, umfasst die Zusammensetzung zumindest ein physiologisch verträgliches Salz eines Metalls und/oder Erdalkalimetalls, wobei das Metall vorzugsweise zweiwertig ist. Da die Xylose-Isomerase in Anwesenheit von Metall- und/oder Erdalkalimetallionen eine gesteigerte Enzymaktivität aufweist, werden entsprechende Salze in der erfindungsgemäßen Zusammensetzung vorgesehen. Es ist insbesondere bevorzugt Xylose-Isomerase mit den oben angeführten Salzen zu ko-kristallisieren. Dadurch können Kofaktoren in das aktive Zentrum des Enzyms in den Kristallen eingebaut werden und liegen somit im Kristallgitter (neben Kristallwasser u.ä.) bereits vor. Die Ionen und Kofaktoren müssen deshalb in der Reaktionslösung (=Darmlumen) nicht mehr zusätzlich vorhanden sein. Erfindungsgemäß kann die Zusammensetzung mindestens 2, mindestens 3, 4, 5, 6, 7, 8, 9, 10 oder sogar mindestens 15 verschiedene Arten von Salzen von Metallen bzw. Erdalkalimetallen umfassen.

Gemäß einer bevorzugten Ausführungsform der vorliegenden Erfindung ist das Erdalkalimetall Magnesium.

Die erfindungsgemäße Zusammensetzung umfasst das Salz des Erdalkalimetalls in der Zusammensetzung in einem molaren Verhältnis zur Xylose-Isomerase von 0,5:1 bis 200:1, vorzugsweise von 5:1 bis 25:1, noch mehr bevorzugt von 12:1 bis 18:1.

Gemäß einer weiteren bevorzugten Ausführungsform der vorliegenden Erfindung ist das Metall Kobalt, Mangan, Zink, Eisen oder Kupfer, wobei dessen Salz in der Zusammensetzung in einem molares Verhältnis zur Xylose-Isomerase von 0,1:1 bis 100:1, vorzugsweise von 0,5:1 bis 20:1, besonders bevorzugt von 3:1 bis 7:1.

Als Anion für diese Salze dient ein pharmazeutisch verträgliches Anion, vorzugsweise ausgewählt aus der Gruppe bestehend aus Chlorid, Sulfat, Carbonat, Hydrogencarbonat oder Maleate.

Gemäß einer besonders bevorzugten Ausführungsform der vorliegenden Erfindung ist das mindestens eine Salz ausgewählt aus der Gruppe bestehend aus MgCl₂, MgSO₄, MgCO₃, Mg(HCO₃)₂, Mg(C₄H₂O₄), CoCl₂, CoSO₄, CoCO₃, Co (HCO₃)₂, Co(C₄H₂O₄), MnCl₂, MnSO₄, MnCO₃, Mn(HCO₃)₂ oder Mn(C₄H₂O₄).

In einer besonders bevorzugten Ausführungsform umfasst die erfindungsgemäße Zusammensetzung Magnesium- und/oder Kobaltsalze. Ganz besonders bevorzugt sind erfindungsgemäß Zusammensetzungen, die sowohl Mg- als auch Co-Salze enthalten. Die Kombination dieser zwei Salze hat sich als besonders geeignet zur Behandlung der Fruktose-Malabsorption erwiesen.

Gemäß einer bevorzugten Ausführungsform der vorliegenden Erfindung werden die Kristalle der Xylose-Isomerase als feines, getrocknetes Pulver verwendet. Das Enzympulver ist gegenüber bakteriellen Abbau stabiler und hat großtechnisch in der Pelletierung Vorteile in der Verarbeitung, wie die bessere Dosierbarkeit und bessere Mischbarkeit.

Das Pulver der Xylose-Isomerase hat bevorzugt einen Restwassergehalt von 0,1% bis 30%, besonders bevorzugt von 0,5% bis 10% und ganz besonders bevorzugt von 1% bis 3%. Der Proteingehalt des Pulver beträgt bevorzugt 50% bis 99,9%, besonders bevorzugt 75% bis 99,9%, ganz besonders bevorzugt 95% bis 99,9%. Die Teilchengröße des Pulvers beträgt 0,01µm bis 1000µm, bevorzugt von 0,1µm bis 100µm und besonders bevorzugt von 1µm bis 30µm.

Die erfindungsgemäß hergestellte und zur Verfügung gestellte Xyloseisomerase-Zusammensetzung ist vorzugsweise "hochaktiv". Diese "hochaktive" Xyloseisomerase-Zusammensetzung weist vorzugsweise eine enzymatische Aktivität von 35.000 bis 45.000 Einheiten pro Gramm (Gesamtpräparat) auf. Dabei ist eine Einheit (Unit (U)) definiert als µmol pro Gramm pro Stunde bei 37°C (35.000 bzw. 45.000 U entsprechen 9,72 bzw. 12,5 milli-Katal (µkat; kat=mol/s). Im Gegensatz dazu weist eine Xyloseisomerase aus einem Lyophilisat bzw. z.B. von einer Säule direkt gereinigt eine Aktivität von ca. 4.000 bis 6.000 U/g (bestimmt nach Dische et al., J. Biol. Chem. (1951)192:583), also 1,1 bis 1,7 mkat auf.

Gemäß einer weiteren bevorzugten Ausführungsform der vorliegenden Erfindung wird eine Magensaft-resistente Darreichungsform ausgewählt aus der Gruppe bestehend aus Magensaft-resistentes Pellet, Magensaft-resistente Tablette, Magensaft-resistente Kapsel, Magensaft-resistentes Granulat, Magensaft-resistente Dragees und Magensaft-resistentes Pulver zur Verabreichung im Menschen eingesetzt und zur Verfügung gestellt.

Erfindungsgemäß können jegliche Arten von Darreichungsformen verwendet werden, sofern diese eine rasche und wirksame Aktivitätsfreisetzung am Zielort sicherstellen. In einer bevorzugten Ausführungsform können die erfindungsgemäßen Darreichungsformen mit wenigstens einem Überzug, besonders bevorzugt mit einem Magensaft-resistenten Überzug, versehen sein. Diese Überzüge werden vorzugsweise in einer Menge von 1 bis 50 Gew.%, bezogen auf das Gesamtgewicht der Darreichungsformen, aufgetragen. Besonders vorteilhaft sind Methacrylsäure/Alkyl(meth)acrylat-Copolymere, besonders bevorzugt Copolymere von Methacrylsäure/Methylmethacrylat mit einem Verhältnis von 1:1 bis 1:2 wie Eudragit L® oder Eudragit S®, ganz besonders bevorzugt Copolymere von Methacrylsäure/Ethylacrylat 1:1, wie Eudragit L55®, Eudragit L30D-55®, die sich bei einem pH-Wert von > 5,5 schnell auflösen. Weiterhin können Magensaft-resistente Überzüge auf Basis von Cellulosen oder auf Basis von Schellack, die dem Fachmann bekannt sind, aufgetragen werden. Die Auftragung der Überzüge kann mit entsprechenden Lösungen oder Dispersionen in organischem oder wässrigem Medium erfolgen, wobei ein wässriges Medium bevorzugt ist. Die Magensaft-resistenten Darreichungsformen sind vorzugsweise auch gegenüber Speichel resistent, wobei sich hierfür vorzugsweise Überzüge auf Basis von Eudragit E oder Eudragit EPO eignen.

Erfindungsgemäß wird unter "Magensaft" sowohl die natürliche Komposition des Magensafts als auch die dem Fachmann bekannten künstlichen Magensaft ähnlichen Zubereitungen (pH 1-2) verstanden. Ebenso werden unter "Freisetzung in Dünndarm" sowohl die Freisetzung im natürlichen Dünndarmsaft als auch die Freisetzung in Dünndarmsaft-ähnlichen Zubereitungen bei pH-Werten von 6-7,5, vorzugsweise pH 6,4-6,8, verstanden.

Erfindungsgemäß wird als "magensaftresistent" die Eigenschaft einer Darreichungsform bezeichnet, die einen darin enthaltenen Wirkstoff (z.B. Xylose-Isomerase) bei Einwirkung eines Magensafts bzw. einer Lösung, die vergleichbare Eigenschaften wie Magensaft aufweist (z.B. Säure), für einen bestimmten Zeitraum von mindestens 10, vorzugsweise mindestens 20, noch mehr bevorzugt mindestens 30, insbesondere mindestens 60, Minuten derart zu schützen vermag, dass der Wirkstoff einen Aktivitätsverlust von maximal 50%, vorzugsweise von maximal 40%, noch mehr bevorzugt von maximal 30%, am meisten bevorzugt maximal 20%, insbesondere maximal 10%, erfährt.

Die bevorzugten Darreichungsformen werden hergestellt, indem man die Ausgangsstoffe mit der erfindungsgemäßen Enzympräparation mischt, granuliert, extrudiert, zerteilt und ggf. formt, vorzugsweise sphäronisiert, ggf. klassiert und mit einem Magensaft-resistenten Überzug versieht.

Magensaft-resistente Pellets sind Pellets, die von einem Magensaft-resistenten Überzug umhüllt sind, die sich bei einem pH-Wert auflösen, wie er im Darmtrakt vorzufinden ist. D.h. derartige Überzüge lösen sich somit vorzugsweise bei einem pH-Wert von mindestens 4 und maximal 10 auf. Eudragit, beispielsweise, ein Magensaft-resistenter Überzug basierend auf anionischen Polymeren von Methacrylsäure und Methacrylaten, enthält -COOH als funktionelle Gruppe und löst sich im Bereich von pH 5,5 bis pH 7 auf. Alternativ zu Eudragit können Schellack oder acetylierte Stärke (z.B. Amprac 01) herangezogen werden. Da die im Stand der Technik bekannten Magensaft-resistenten Überzüge verschiedene Eigenschaften aufweisen (z.B. pH-Wert, bei dem sich der Überzug auflöst, Auflösgeschwindigkeit) können die Materialien der Überzüge auch kombiniert werden. Schellack, beispielsweise, zeigt eine gute Säureresistenz, löst sich allerdings sehr langsam im Darmtrakt auf. Amprac 01 hingegen löst sich rasch im Darmmilieu auf, zeigt jedoch keine ausreichende Säureresistenz. Um die Nachteile eines Materials zu kompensieren, können die beiden oben genannten Materialien beispielsweise in einem Gewichtsverhältnis von 60-95/40-5, vorzugsweise von 70-90/30-10, Schellack/Amprac 01 gemischt werden. Ein weiterer Parameter, der die Freisetzungsgeschwindigkeit des Wirkstoffs beeinflusst, ist die Schichtdicke des Magensaft-resistenten Pellets. Die Schichtdicke, ausgedrückt als Massenverhältnis, ist dabei vorzugsweise 5 bis 30%, noch mehr bevorzugt 10 bis 20% der Gesamtmasse des Endprodukts. Die Pellets weisen vorzugsweise einen durchschnittlichen Durchmesser von 0,5 bis 5 mm, insbesondere von 0,7 bis 2 mm, auf. Eine derartige Größe hat den Vorteil, dass die Pellets den Magen schnell passieren können.

Die Herstellung der Pellets, die den Einsatz der erfindungsgemäßen Enzympräparation als Arzneimittel, Nahrungsergänzungsmittel, diätetisches Lebensmittel, Medizinprodukt, Futtermittel, Ergänzungsfuttermittel oder diätetisches Futtermittel erlaubt, erfolgt vorzugsweise mit einem Extruder, der eine thermische Stabilität der Inhaltsstoffe der Zusammensetzung, insbesondere der Enzyme, von bis zu 60°C erforderlich macht (Stricker Arzneiformenentwicklung, Springer Verlag 2003). Die Pellets können neben einem Magensaft-resistenten Überzug und den Enzymen weitere pharmazeutische Zusatzstoffe umfassen. Beispielsweise dient mikrokristalline Zellulose (z.B. Avicel) als Füllstoff und Quellstoff. Zellulose ist unlöslich in Wasser, hat in dieser Form sowohl kristalline als auch amorphe Anteile. Diese Kombination bewirkt eine plastische Verformbarkeit, d.h., dass bei genügend hoher Kraft eine irreversible Formveränderung eintritt. Dies ist eine wesentliche Voraussetzung für das Pelletieren im Extruder und Spheronizer. Bei der feuchten Granulierung nimmt mikrokristalline Zellulose große Wassermengen auf und wird dadurch auch ohne Bindemittelzusatz zu einer gut komprimierbaren, zusammenhaltenden Masse. Die Menge an mikrokristalliner Zellulose in einem Pellet kann erfindungsgemäß zwischen 5 und 70%, vorzugsweise zwischen 10 und 60%, noch mehr bevorzugt zwischen 15 und 50%, betragen. Als Binde- und Füllmittel kann Maltose verwendet werden. Maltose erhöht die Löslichkeit der Matrix und unterstützt damit die rasche Freisetzung des Enzyms. Maltose kann erfindungsgemäß zu 1 bis 40%, vorzugsweise 5 bis 35%, noch mehr bevorzugt 10 bis 30%, einem Pellet zugesetzt werden. Maltose hat gegenüber der üblicherweise verwendeten Saccharose den Vorteil, dass es keine Fruktose aufweist und somit nicht unnötige und wiederum schädliche Fruktose in den Körper einbringen kann.

Hydroxypropylcellulose (zugesetzt in einer Menge von vorzugsweise 0,5 bis 10%) kann ebenfalls als Bindemittel zugesetzt werden und dient zur Verhinderung von Feinstaub. Ferner erhöht Hydroxypropylcellulose die Festigkeit der Pellets und trägt somit wiederum zur Verbesserung der Ausbeute bei. Stärke kann als Füll- und Sprengmittel dem erfindungsgemäßen Pellet zugesetzt werden (in einer bevorzugten Menge von 1 bis 30%). Als wasserunlösliche Substanz kann Stärke viel Wasser aufnehmen und ist daher ein ideales Sprengmittel. Crosscarmellose (Na-CMC; Acdisol) ist ein reines Sprengmittel, das vorzugsweise in einer Menge zwischen 1% und 5% eingesetzt werden kann. Ein zu hoher Anteil an Acdisol führt zu frühem Zerfall der Pellets bereits beim Ausrunden und ist daher kontraproduktiv. Crosspovidon, ein quervernetztes PVP, ist ebenfalls wasserunlöslich und dient ebenfalls als Sprengmittel. Aufgrund seiner Polymereigenschaften unterstützt es die bessere Ausrundung bei der Herstellung von Pellets (kann vorzugsweise in einer Menge von 0,5 bis 10% zugesetzt werden). Povidon ist ein wasserlöslicher Zusatzstoff und dient als Bindemittel. Die Kombination dieser verschiedenen Füll-, Sprengund Bindemittel führt zu einer molekulardispersen Verteilung der Xylose Isomerase im Pellet und sichert eine rasche Bioverfügbarkeit.

Zwischen dem Magensaft-resistenten Überzug und dem Pellet mit dem Wirkstoff kann eine Isolierschicht aus Glycerin und/oder Talkum vorgesehen sein. Glycerin dient als Feuchthaltemittel, um eine Dehydrierung und damit Inaktivierung des Enzyms zu verhindern.

Da Partikel mit einem Durchmesser größer als 3 mm am Pylorus einen Verschlussreflex auslösen, ist es besonders bevorzugt, dass die Magensaft-resistenten Darreichungsformen, insbesondere Pellets, den Magen in einer Größe von weniger als 3 mm verlassen, da derartige Partikel den Pylorus in geschlossenem Zustand passieren können und wie Flüssigkeit aus dem Magen in den Dünndarm weitertransportiert werden. Der dort vorhandene neutrale pH-Wert bringt die Pellets innerhalb von ca. 5 bis 30 min, vorzugsweise von 15 min, zum Platzen und setzt somit die wirksamen Substanzen frei. Daher ist es besonders bevorzugt als Magensaft-resistente Darreichungsform Pellets mit weniger als 5 mm, vorzugsweise mit weniger als 3 mm, Durchmesser vorzusehen.

Alternativ zu Pellets kann die Xylose-Isomerase auch in Kapseln oder einer sonstigen Darreichungsform durch den Magen in den Darmtrakt transportiert werden. Geeignete Kapseln sind dabei z.B. Gelatine-Kapseln oder Stärkekapseln. Die Kapseln können auch die erfindungsgemäßen Pellets beinhalten.

Gemäß einer besonders bevorzugten Ausführungsform der vorliegenden Erfindung liegt die Xylose-Isomerase in Mikrokapseln, Nanopartikel oder Liposomen vor.

Gemäß einer bevorzugten Ausführungsform ist die Xylose-Isomerase mikrobiellen, tierischen, pflanzlichen oder rekombinanten Ursprungs.

Die erfindungsgemäß verwendeten Enzyme können verschiedensten Ursprungs sein. Verfahren, wie die Enzyme isoliert und/oder hergestellt werden können, sind dem Fachmann hinreichend bekannt.

Es ist insbesondere bevorzugt, Xylose-Isomerase mikrobiellen Ursprungs zu verwenden, die aus einem Mikroorganismus der Familie der Streptomycetaceae, insbesondere Streptomyces rubiginosus, stammt. Xylose-Isomerase aus derartigen Quellen weisen eine höhere spezifische Aktivität auf Glukose/Fruktose und kleinere Km als Isomerasen anderer Quellen auf. So hat die Xylose-Isomerase im Lacotbacillus brevis einen Km von 920mM, Str.rub. einen Km von 160mM.

Die erfindungsgemäße Zusammensetzung kann in Form von verschiedensten Produkten eingesetzt werden. Vorzugsweise ist die Zusammensetzung eine pharmazeutische Zusammensetzung, ein Nahrungsergänzungsmittel, ein diätetisches Lebensmittel, ein Medizinprodukt, ein Futtermittel, ein Ergänzungsfuttermittel oder ein diätetisches Futtermittel.

Die erfindungsgemäße Zusammensetzung wird vorzugsweise zur Behandlung von Fruktose-Malabsorption verwendet.

Ein weiterer Aspekt der vorliegenden Erfindung betrifft die Verwendung von Xylose-Isomerase (EC 5.3.1.5) zur Herstellung eines Arzneimittels zur Behandlung von Fruktose-Malabsorption, wobei die Xylose-Isomerase in wie oben beschriebener kristalliner Form in Arzneimittel eingesetzt wird. Das Arzneimittel kann z.B. in einer Magensaft-resistenten Darreichungsform, wie in der vorliegenden Erfindungsbeschreibung definiert, vorliegen.

Im Falle der oralen Anwendung sollte das Arzneimittel mit der erfindungsgemäßen Xylose-Isomerase vom Konsumenten unmittelbar vor oder zum Genuss fruktosehältiger Nahrungsmittel eingenommen werden, um eine rasche Isomerisierung zu gewährleisten. Da Partikel mit einem Durchmesser größer als 3 mm am Pylorus einen Verschlussreflex auslösen, ist es bevorzugt, dass die Magensaft-resistenten Darreichungsformen den Magen in einer Größe von weniger als 3 mm verlassen, da derartige Partikel den Pylorus in geschlossenem Zustand passieren können und wie Flüssigkeit aus dem Magen in den Dünndarm weitertransportiert werden. Der dort vorhandene neutrale pH-Wert bringt beispielsweise Pellets innerhalb von ca. 5 bis 30 min, vorzugsweise von 15 min, zum Platzen und setzt somit die Xylose-Isomerase frei.

Ein weiterer Aspekt der vorliegenden Erfindung betrifft ein Verfahren zur Herstellung einer Zusammensetzung gemäß der vorliegenden Erfindung umfassend den Schritt des Kristallisierens von Xylose-Isomerase.

Das erfindungsgemäße Verfahren, insbesondere das Kristallisieren der Xylose-Isomerase, wird vorzugsweise in Gegenwart zumindest eines Salzes eines Erdalkalimetalls und/oder Metalls durchgeführt.

Vorzugsweise wird die kristalline Xylose-Isomerase getrocknet und gegebenenfalls pulverisiert. Die Trocknung erfolgt vorzugsweise im Vakuum und gliedert sich in a) Abfiltrieren der

Kristalle aus der Lösung und anschließend b) Gefriertrocknen des Filterkuchens. Der trockene (Restfeuchte < 5%) Filterkuchen wird vorzugsweise anschließend gemahlen, beispielsweise mit einer Getreidemühle.

Gemäß einer bevorzugten Ausführungsform der vorliegenden Erfindung wird die kristalline Xylose-Isomerase als pharmazeutische Darreichungsform, insbesondere ausgewählt aus der Gruppe bestehend aus Magensaft-resistentes Pellet, Magensaft-resistente Tablette, Magensaft-resistente Kapsel, Magensaft-resistentes Granulat und Magensaft-resistentes Pulver formuliert.

Ein weiterer Aspekt der vorliegenden Erfindung betrifft die Zusammensetzung erhältlich nach einem Verfahren gemäß der vorliegenden Erfindung.

Die vorliegende Erfindung wird anhand der folgenden Figuren und Beispiele näher dargestellt, ohne jedoch auf diese beschränkt zu sein.
Fig. 1 zeigt den Einfluss des pH-Wertes auf die Aktivität von Xylose-Isomerase.
Fig. 2 zeigt die Säurestabilität von Xylose-Isomerase. Bei Bedingungen von pH < 4 wird die Enzymaktivität irreversibel zerstört.
Fig. 3 zeigt die Temperaturstabilität der Aktivität der erfindungsgemäßen hochaktiven Xylose-Isomerase in der bevorzugten Ausführungsform von magensaftresistent überzogenen Pellets.
Fig. 4 zeigt einen Vergleich der Aktivität von über einen Kristallisationsprozess gereinigter Xylose-Isomerase zu isolierter Xylose-Isomerase.
Fig. 5 zeigt den Einfluss von Ionen zweiwertiger Metalle auf die Aktivität von Xylose-Isomerase.
Fig. 6 zeigt den Einfluss der Kokristallisation der Xylose-Isomerase mit zweiwertigen Metallionen auf die Aktivität der Xylose-Isomerase.

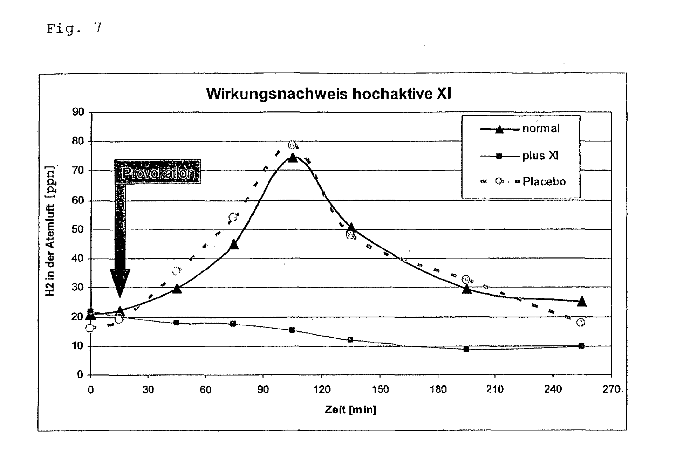

Fig. 7 zeigt die in vivo Wirkung von Xylose-Isomerase über die Zeit im Vergleich zu einem Placebo und keiner Gabe eines Mittels.

### BEISPIELE:

### Beispiel 1:

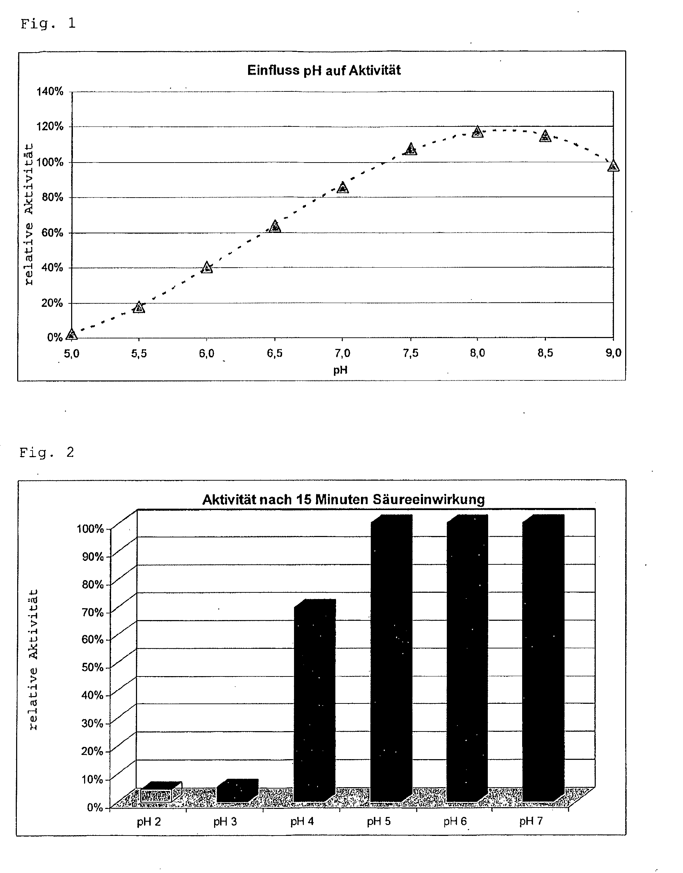

Um eine physiologisch wirksame Bioverfügbarkeit des Enzyms im Darmbereich eines Individuums oder Tiers sicherzustellen, ist es von Vorteil Xylose-Isomerase Magensaft-resistent geschützt in den Dünndarm zu transportieren. Basis zur Entwicklung einer entsprechenden Rezeptur ist die Feststellung der pH-Abhängigkeit der Aktivität des Enzyms (Fig. 1) und die Stabilität des Enzyms bei bestimmten pH-Werten (Fig. 2). Die pH-Abhängigkeit der Aktivität der Glucose Isomerase wurde in 50mM Maleat, Hepes oder Tris-Buffer pH 5 bis pH 9 mit 5mM MgSO₄, 1mM CoCl₂ und 100mM Glukose bei 37°C gemessen. Die enstandene Fruktose wurde mit einem modifizierten Schwefelsäure/Carbazol-Test nach Dische und Bohrenfreud (Dische, Z et al.:J Biol Chem.(1951) 192 : 583) gemessen. Für die Stabilität wurde eine definierte Menge Enzym für 30' in einen 50mM Glycin, Maleat oder Hepes-Puffer mit einem pH von 2 bis 7 bei 37°C inkubiert. Der Buffer wurde mit einem Überschuss 100mM Hepes-Buffer pH 7,4 neutralisiert und die Aktivität in Gegenwart von 5mM MgSO₄, 1mM CoCl₂ und 100mM Glukose bei 37°C gemessen. Die enstandene Fruktose wurde mit einem modifizierten Schwefelsäure/Carbazol-Test nach Dische et al. gemessen.

### Beispiel 2:

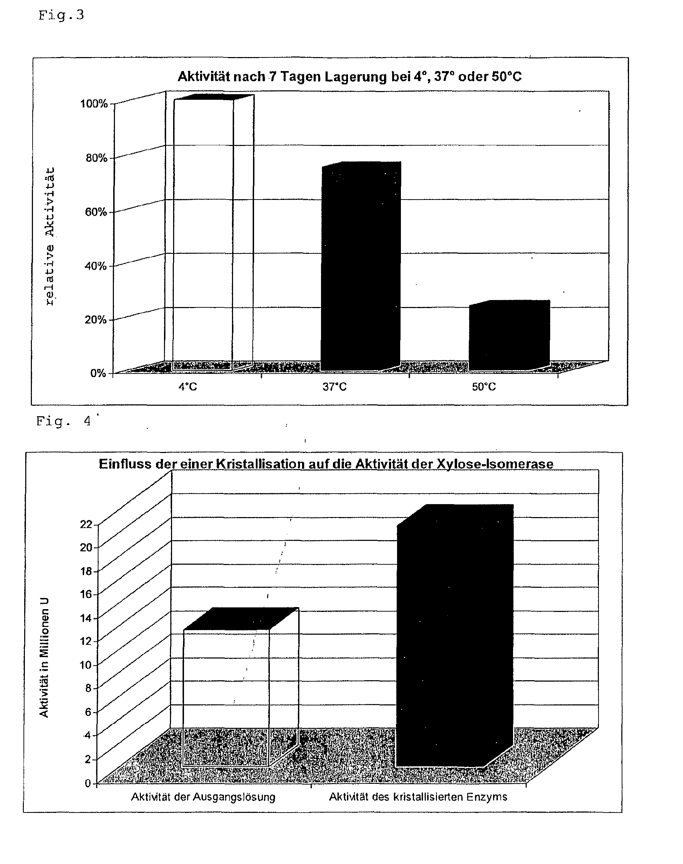

Temperaturstabilität der Aktivität der erfindungsgemäßen hochaktiven Xylose-Isomerase in der bevorzugten Ausführungsform von magensaftresistent überzogenen Pellets. Die Stabilität der Enzympellets ist für die Lagerung und den Verkauf von großer Bedeutung. Eine ausreichende Stabilität der Aktivität muss erreicht werden, um eine für den Handel aktzeptierbare Haltbarkeit vorweisen zu können. Überzogene Pellets wurden 7 Tage bei 4°C, 37°C und 50°C gelagert und die Aktivität unter Standardbedingungen gemessen. Die Aktivität nahm nach 7 Tagen auf 37°C um 25% und nach 7 Tagen auf 50°C um 77% ab (Fig. 3).

### Beispiel 3:

Vergleich der Aktivität von über einen Kristallisationsprozess gereinigter Xylose-Isomerase zu isolierter Xylose-Isomerase.

Großtechnisch wurde Xylose-Isomerase aus *Streptomyces rubiginosus* gewonnen. Diese erwerbbare Xylose-Isomerase wurde durch einen Kristallisationsprozess von Sorbitol und weiteren Zusätzen gereinigt. Dadurch konnte eine Aktivitätssteigerung von 70 % erreicht werden. Die Aktivität der in Beispiel 1 gewonnenen Kristall-Suspension und die Aktitvität der Ausgangslösung wurde unter Standardbedingungen gemessen. Dabei ergab sich eine durchschnittliche Aktivitätssteigerung um 70% (Fig. 4).

### Beispiel 4: Einfluss von Ionen zweiwertiger Metalle auf die Aktivität von Xylose-Isomerase.

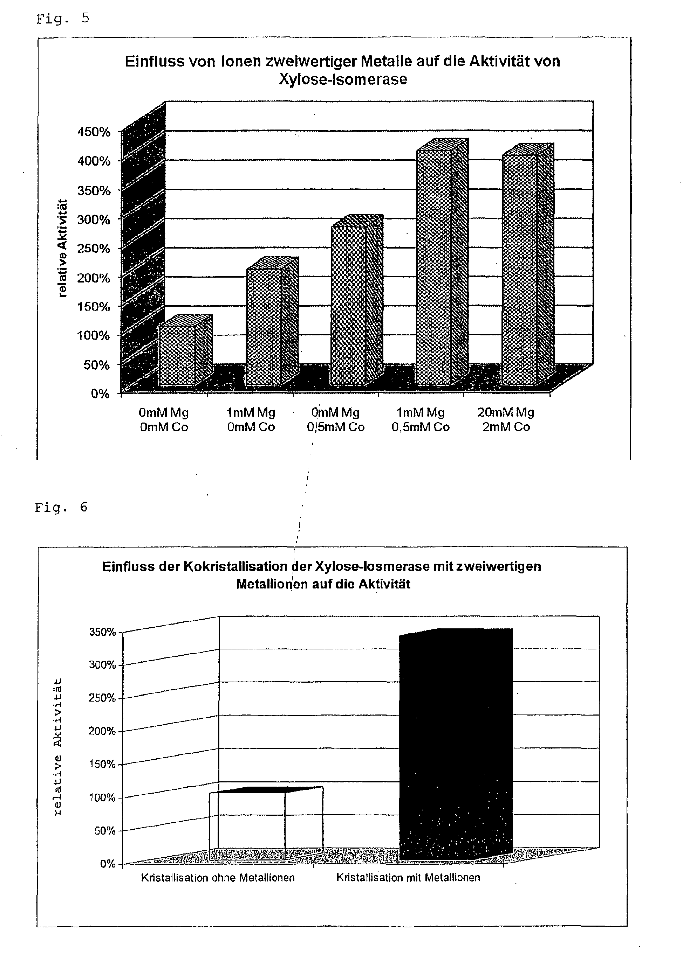

Eine ohne Zusatz von zweiwertigen Metallionen gereinigte Xylose Isomerase wurde mit unterschiedlichen Konzentrationen an Mg²⁺ und Co²⁺ versetzt und die Aktivität in 50mM Phosphat-Buffer pH7,4 und 100mM Glukose bei 37°C gemessen. Dabei ergab sich eine Aktivitätssteigerung von 300% (Fig. 5).

### Beispiel 5:

Einfluss der Kokristallisation der Xylose-Isomerase mit zweiwertigen Metallionen auf die Aktivität der Xylose-Iosmerase.

Xylose-Isomerase wurde wie in Beispiel 1 mit oder ohne Mg²⁺ und Co²⁺ kristallisiert und die Aktivität in 50mM Phosphat-Buffer pH7,4 ohne zweiwertige Metallionen mit 100mM Glukose bei 37°C gemessen. Dabei ergab sich eine spezifische Aktivität der Kristalle mit zweiwertigen Metallionen von 300% verglichen mit Kristallen ohne eingebauten Metallionen (Fig. 6).

### Beispiel 6: Kristallisation der Xylose-Isomerase in Gegenwart von Kofaktoren

5l einer 4 w/v % Xylose-Isomerase Lösung werden mit 735g Ammoniumsulfat, 72g Magnesiumsulfat-Hexahydrat und 19,4g Cobalt(II)chlorid-Hexaydrat versetzt und langsam auf 2°C abgekühlt und 20h gerührt. Zur Beschleunigung des Kristallisationprozesses können 50ml einer 4% Xylose-Isomerase Kristallsuspension hinzugefügt werden. Die enstandenen Kristalle sollten eine optimale Größe zwischen 50µm und 100µm aufweisen.

Diese Vorgehensweise entspricht ungefähr der Kristallisation in US 4,699,822, allerdings wird hier CoCl₂ und die Starterkristalle zugesetzt.

### Beispiel 7: Trocknung der Xylose-Isomerase-Kristalle

Die in Beispiel 1 gewonnene Xylose-Isomerase-KristallSuspension wird durch einen Faltenfilter Klasse 3hw (Sartorius, Germany) filtriert. Die gewonnenen Kristalle werden gefroren und lyophilisert. Zur optimalen Verarbeitbarkeit wird der feste Enzymkuchen fein gemahlen. Das entstandene Xylose-Isomerase-Pulver hat typischerweise eine Aktivität von 45000 Units pro Gramm. Eine Einheit an Xylose-Isomerase ist definiert als die Enzymaktivität, die in 50mM Phosphatbuffer pH7,4 mit 5mM MgSO₄, 1mM CoCl₂ und 100mM Glukose bei 37°C (Standardbedingungen) pro Stunde 1 µmol (180 µg) Glukose in Fruktose umwandelt.

### Beispiel 8: Herstellung von Xylose-Isomerase Pellets

### a) Granulierung:

31,4 g Hydroxypropylcellulose, 408,7 g mikrokristalline Cellulose, 169,5 g Reisstärke, 15,6 g Croscarmellose, 62,2 g Crospovidon, 145,3g Maltose werden mit 167,1g Xylose-Isomerase-Pulver (7.500.000 Units) gemischt. Die Feststoffmischung wurde mit 377g bidestilliertem Wasser zu einer feuchten, krümeligen Masse verarbeitet. Diese Masse wurde über ein Sieb mit einer Porenweite von 1 mm zu Strängen extrudiert (Caleva Extruder 10/25, Caleva Process Solutions Ltd.)

### b) Sphäronisierung:

Die feuchten Stränge wurden in einem Spheronizer (Spheronizer 250, Caleva Process Solutions Ltd.) bei 400 Upm 5 Minute lang zu Pellets rolliert. Danach wurden die Pellets bei 35°C bis zur Gewichtskonstanz auf Horden getrocknet.

### c) Klassierung:

Die getrockneten Pellets wurden in einem Siebturm klassiert, wobei die Fraktion im Größenbereich von 0,4 bis 0,8 mm zur Weiterverarbeitung geeignet ist.

### d) Charakterisierung der Pellets:

Die Gesamtausbeute betrug 70%.

Die Freisetzungsuntersuchung im Dissolutionstester führte zu einer Freigabe von 85,8% der Aktivität innerhalb von 5 Minuten und 97,5% der eingebrachten Aktivität innerhalb von 15 Minuten in die unmittelbare Umgebung. Durch diese rasche Freisetzung des Enzyms innerhalb kurzer Zeit wird eine rasche Wirkung im Gastrointestinaltrakt nach peroraler Einnahme erreicht.

### e) Überziehen der Pellets:

Aufgrund seiner Proteinstruktur wird Xylose-Isomerase im Magen durch Pepsin und sauren pH-Wert weitgehend inaktiviert. Damit ist ein Schutz des Enzyms durch einen Magensaft-resistenten Überzug bzw. eine Magensaft-resistente, anionische Matrix eine Voraussetzung für die Erhaltung der Enyzmaktivität.

Überzugslösung: 1,9 kg acetylierte Stärke wurden in 15,2 kg gereinigtem Wasser unter Rühren gelöst (Suspension 1). In weiteren 100 kg gereinigtem Wasser wurden 10,9 kg Schellack SSB 63 Hydram mit einem Ultraturrax bis zur Lösung gerührt (Suspension 2). Die Suspensionen 1 und 2 wurden vermischt und 0,6 kg Glycerol 85% und 2,6 kg mikronisierter Talkum zugesetzt. Während des Sprühvorganges wurde die Überzugslösung mit einem Ultraturrax gerührt.

Überziehen: 1 kg Pellets wurden mit 1,5 kg Überzugslösung im Wirbelschichter überzogen. Die Geräteparameter wurden wie folgt gewählt: Sprühdruck 1,6 bar, Sprührate 180 g/Minute, Zulufttemperatur 55°C, Produkttemperatur: 35°C; Zuluftmenge 1400 m³/h.

Nach Auftragen der gesamten Sprühlösung wurden die Pellets 60 Minuten bei einer Zulufttemperatur von 40°C nachgetrocknet.

### g) Charakterisierung des Produktes:

Die überzogenen Pellets entsprachen der Prüfung der Magensaftresistenz laut Pharmakopoea Europaea. Nach zweistündiger Inkubation im Zerfallstester bei 37°C in 0,1 N Salzsäure waren die Pellets unverändert, ein Austausch des Mediums auf Phosphatpuffer pH 6,8 führte innerhalb von einer Stunde zum Zerfall der Magensaft-resistent überzogenen Pellets. Der Überzug machte etwa 16% des Ursprungsgewichtes aus. Die Pellets konnten auch als Vorprodukt für Tabletten und Kapseln verwendet werden.

### Beispiel 9: Sprühtrocknung von Xylose-Isomerase

### a) Herstellung der Sprühlösung:

5 g Celluloseacetatphtalat wurden zu 80 g Wasser gegeben und unter Rühren mit wässriger 25-prozentiger Ammoniaklösung das Celluloseacetatphtalat gelöst und der pH-Wert auf 7,5 eingestellt. Die Lösung wurde mit 5 g einer wässrigen 100 mM MgSO₄-Lösung versetzt und Xylose-Isomerase-Kristallsuspension, die einer Trockenmasse an Enzym von 1,25 g entsprach, zugesetzt.

### b) Sprühtrocknen:

Folgende Einstellungen wurden verwendet: Inlettemperatur 130°C, Outlettemperatur 90°C, Pumpgeschwindigkeit 1,5 ml/min, 800 l/h Druckluft und -40 mbar Aspiration.

### c) Charakterisierung des Produktes:

Die Produktausbeute betrug 50%. Die Aktivität blieb zu 90% erhalten. Das Xylose-Isomerase-Pulver entspricht der Prüfung der Magensaftresistenz laut Pharmakopoea Europaea. Nach zweistündiger Inkubation im Zerfallstester bei 37°C in 0,1 N Salzsäure war das Pulver unverändert, ein Austausch des Mediums auf Phosphatpuffer pH 6,8 führte innerhalb von einer Stunde zu einer kompletten Lösung des Pulvers. Allerdings konnte in dieser Lösung nur mehr 5% der ursprünglichen Enzymaktivität gemessen werden. Dies lässt den Schluss zu, dass zwar die makroskopische Struktur des Pulvers erhalten geblieben ist (was die Pharm Eur fordert), die Enzymaktivität aber verloren gegangen ist (was kein Kriterium der Pharm Eur ist)

### Beispiel 10: Symptomatische Wirksamkeit

In einer klinischen Studie wurden Probanden mit einer diagnostizierten Fruktose-Malabsorption angehalten, in einem Fragebogen die subjektiven Beschwerden nach einer bestimmten, fruktosehältigen Mahlzeit festzuhalten. Im Abstand von etwa 2 Tagen sollte dieselbe Mahlzeit gemeinsam mit einer steigenden Anzahl von Xylose-Isomerase-hältigen Kapseln gegessen werden und der Symptomverlauf dokumentiert werden.

Überraschenderweise führte bereits die Zufuhr einer Kapsel (= 920 Units Xylose-Isomerase) pro Mahlzeit zu einer deutlichen Verbesserung der Symptome, die Einnahme von 2 Kapseln führte bei 75% der Probanden zu einem völligen Schwinden der Symptome. Bei weiteren 13% wurden die Symptome verringert.

Die durchschnittlich aufgenommene Menge Fruktose pro Mahlzeit in der klinischen Studie betrug etwa 7 g. Ein Unit Xylose-Isomerase ist definiert als Enzymmenge, die bei pH 7,4 pro Stunde 1 µmol (180 µg) Glukose in Fruktose (und umgekehrt) umwandelt. Das bedeutet, dass bei einer durchschnittlichen Dünndarmtransitzeit von einer Stunde 2 Kapseln insgesamt 0,33 g Fruktose unter Standardbedingungen im Labor abbauen. Dieser im Labor erreichte Wert liegt weit unter der bei der klinischen Studie aufgenommenen Menge von 7 g.

Durch die spezielle Darreichungsform von hochaktiver Xylose-Isomerase kann daher die Verträglichkeit von Fruktose dramatisch verbessert und "Short bowel syndrom" (Kurzdarmsyndrom) und verwandte Krankheitsbilder erfolgreich therapiert werden.

### Beispiel 11: Klinische Wirksamkeit

5 Personen, welche auf die Provokation von 25 Gramm Fruktose in 100 ml Wasser mit einer signifikanten Erhöhung des Wasserstoffgasgehaltes in der Atemluft reagierten, wurden unmittelbar vor der Provokation 3 Kapseln der erfindungsgemäßen Präparation verabreicht. Während der darauffolgenden 4 Stunden konnte überraschenderweise überhaupt kein Anstieg der H2-Konzentration in der Atemluft festgestellt werden. Die Verabreichung von Placebo-Kapseln zeigte keinerlei Wirkung auf die Produktion von Wasserstoffgas in der Atemluft. Diese Daten belegen, dass mit der Nahrung aufgenommene Fruktose von der erfindungsgemäßen hochaktiven Xyloseisomerasepräparation während der Passage durch den Dünndarm so effizient in Glukose umgewandelt wird, dass keine physiologisch relevanten Mengen der Fruktose in den Dickdarmbereich vordringen kann. 25 g Fruktose entsprechen in etwa der Menge, die mit der Nahrung pro Tag aufgenommen wird. Daher wurden auch 3 Kapseln der erfindungsgemäßen Präparation verabreicht.

## Patentansprüche

1. Zusammensetzung umfassend kristalline Xylose-Isomerase (EC 5.3.1.5) erhalten durch die Co-Kristallisation mit einem Magnesiumsalz und zumindest einem zweiwertigen Salz ausgewählt aus der Gruppe der Metallsalze, bestehend aus Kobalt, Mangan, Zink, Eisen und Kupfer, wobei die mit zweiwertigen Metallionen co-kristallisierte Xylose-Isomerase eine Enzymaktivitätssteigerung von etwa 300% aufweist.

2. Zusammensetzung nach Anspruch 1, **dadurch gekennzeichnet, dass** das Magnesiumsalz in der Zusammensetzung in einem molaresVerhältnis zur Xylose-Isomerase von 0,5:1 bis 200:1, vorzugsweise von 5:1 bis 25:1, noch mehr bevorzugt von 12:1 bis 18:1, enthalten ist.

3. Zusammensetzung nach einem der Ansprüche 1 bis5, **dadurch gekennzeichnet, dass** das Metallsalz in der Zusammensetzung in einem molares Verhältnis zur Xylose-Isomerase von 0,1:1 bis 100:1, vorzugsweise von 0,5:1 bis 20:1, besonders bevorzugt von 3:1 bis 7:1, enthalten ist.

4. Zusammensetzung nach einem der Ansprüche 1 bis 6, **dadurch gekennzeichnet, dass** das mindestens eine Salz ausgewählt ist aus der Gruppe bestehend aus MgCl₂, MgSO₄, MgCO₃, Mg(HCO₃)₂, Mg(C₄H₂O₄), CoCl₂, CoSO₄, CoCO₃, Co(HCO₃)₂, Co(C₄H₂O₄), MnCl₂, MnSO₄, MnCO₃, Mn(HCO₃)₂ und Mn(C₄H₂O₄).

5. Zusammensetzung nach einem der Ansprüche 1 bis 4, **dadurch gekennzeichnet, dass** diese in einem Magensaft-resistenten Darreichungsform vorliegt, die insbesondere ausgewählt ist aus der Gruppe bestehend aus Magensaft-resistentes Pellet, Magensaft-resistente Tablette, Magensaftresistente Kapsel, Magensaft-resistentes Granulat und Magensaft-resistentes Pulver.

6. Zusammensetzung nach einem der Ansprüche 1 bis 5, **dadurch gekennzeichnet, dass** die Xylose-Isomerase in Mikrokapseln, Nano-partikel oder Liposomen vorliegt.

7. Zusammensetzung nach einem der Ansprüche 1 bis 4, **dadurch gekennzeichnet, dass** die Xylose-Isomerase mikrobiellen, tierischen, pflanzlichen oder rekombinanten Ursprungs ist.

8. Zusammensetzung nach Anspruch 10, **dadurch gekennzeichnet, dass** die Xylose-Isomerase mikrobiellen Ursprungs aus einem Mikroorganismus der Familie der Streptomycetaceae, insbesondere Streptomyces rubiginosus, stammt.

9. Zusammensetzung nach einem der Ansprüche 1 bis 8, **dadurch gekennzeichnet, dass** die Zusammensetzung eine pharmazeutische Zusammensetzung, ein Nahrungsergänzungsmittel, ein diätetisches Lebensmittel, ein Medizinprodukt, ein Futtermittel, ein Ergänzungsfuttermittel oder ein diätetisches Futtermittel ist.

10. Verwendung von Xylose-Isomerase (EC 5.3.1.5) zur Herstellung eines Arzneimittels zur Behandlung von Beschwerden, die mit einer erhöhten Fructoseaufnahme einhergehen, wobei die Xylose-Isomerase in kristalliner Form in einer Zusammensetzung nach einem der Ansprüche 1 bis 9 vorliegt.

11. Verfahren zur Herstellung einer Zusammensetzung gemäß einem der Ansprüche 1 bis 9 umfassend den Schritt des Kristallisierens von Xylose-Isomerase in Gegenwart zumindest eines Salzes eines Erdalkalimetalls und eines zweiwertigen Metallsalzes ausgewählt aus der Gruppe bestehend aus Kobalt, Mangan, Zink, Eisen und Kupfer, wobei die kristalline Xylose-Isomerase getrocknet und gegebenenfalls pulverisiert und als pharmazeutische Darreichungsform formuliert wird.
